(19) Europäisches Patentamt

European Patent Office

Office européen des brevets

(11) **EP 0 286 903 B2**

(12) NEW EUROPEAN PATENT SPECIFICATION

(45) Date of publication and mention
of the opposition decision:
**29.03.2000 Bulletin 2000/13**

(45) Mention of the grant of the patent:
**03.06.1992 Bulletin 1992/23**

(51) Int. Cl.[7]: **A61K 31/557**
// (A61K31/557, 31:535),
(A61K31/557, 31:135)

(21) Application number: **88104999.3**

(22) Date of filing: **28.03.1988**

(54) **Use of a prostaglandin in combination with an adrenergic blocking agent for reduction of intraocular pressure**

Anwendung eines Prostaglandins in Mischung mit einem adrenergischen Blocker zur Verminderung des Augen-Innendruckes

Application d'une prostaglandine en mélange avec un bloqueur adrénergique pour réduire la pression intra-oculaire

(84) Designated Contracting States:
**AT BE CH DE ES FR GB GR IT LI LU NL SE**

(30) Priority: **03.04.1987 US 34484**

(43) Date of publication of application:
**19.10.1988 Bulletin 1988/42**

(73) Proprietors:
• **THE TRUSTEES OF COLUMBIA UNIVERSITY IN THE CITY OF NEW YORK**
**New York, New York 10027 (US)**
• **Pharmacia & Upjohn Aktiebolag**
**112 87 Stockholm (SE)**

(72) Inventors:
• **Bito, Laszlo Z.**
**New York, New York 10033 (US)**
• **Stjernschantz, Johan Wilhelm**
**S-752 38 Uppsala (SE)**

(74) Representative:
**Hansen, Bernd, Dr. Dipl.-Chem.**
**Hoffmann Eitle,**
**Patent- und Rechtsanwälte,**
**Postfach 81 04 20**
**81904 München (DE)**

(56) References cited:
**EP-A- 0 242 580**          **US-A- 4 599 353**

• **CHEMICAL ABSTRACTS, vol. 87, no. 11, Sept. 12, 1977, p. 52, ref.no. 78552x; Columbus, Ohio, US; T.J.ZIMMERMANN et al.:"Timolol. Dose response and duration of action"**
• **CHEMICAL ABSTRACTS, vol. 96, no. 5, Febr. 1, 1982, page 69, ref.no. 28640q; Columbus, Ohio, US; H.J.MERTE:"Intraocular pressure and beta-receptor blockers"**
• **Opthalmic Res. 15, pp. 160-167 (1983)**
• **Investigative Opthalmology and Visual Science, vol. 25, pp. 1087-1093 (1984)**
• **Arch Ophthalmol, vol. 95, pp. 605-607 (1977)**
• **Ophthalmology, vol. 92, no. 9, pp. 1271-1276 (1985)**
• **Albrecht von Graefes Arch Klin Ophthalmol, vol. 217, pp. 175-181 (1981)**
• **Osaka Ika Daigaku Zasshi, vol. 45(1), pp. 1-7 (1986), (C.A. 106:27330b)**
• **Augenheilkunde by Leydhacker, 23rd Edition, Springer Verlag, 1987, pp. 165-171**
• **Arch Ophthalmol, vol. 99, pp. 1212-1216 (July 1981)**

Remarks:
The file contains technical information submitted after the application was filed and not included in this specification

EP 0 286 903 B2

## Description

### Background Of The Invention

The invention described herein was made with government support under grant number EY00333 from the National Eye Institute, National Institutes of Health, Department of Health and Human Services. The U.S. Government has certain rights in this invention.

Glaucoma, an eye disorder afflicting various mammals, including primates, is characterized by increased intraocular pressure (ocular hypertension). In man, such ocular hypertension results from an imbalance between the rate of secretion of aqueous humor by the ciliary epithelium into the posterior chamber of the eye and the resistance to drainage of the aqueous humor from the anterior chamber, primarily via the canal of Schlemm. It is generally believed that increased outflow resistance due to obstruction of aqueous humor drainage routes is the primary cause of the imbalance.

Chronic glaucoma typically results in slow, progressive loss of visual fields and, if not controlled, ultimately in blindness. Initial treatment usually involves topical application of agonists or antagonists of autonomic neuroeffectors, particularly pilocarpine or timolol. If treatment with such topically applied drugs is not effective, systemic administration of carbonic anhydrase inhibitors may be employed. If such approaches are unsuccessful, the glaucoma may have to be treated by surgery or laser.

Eicosanoids and their derivatives include numerous biologically useful compounds. For example, the prostaglandin (PG) group, naturally occurring cyclic fatty acids, is known to possess diverse biological activities. Originally isolated as lipid-soluble extracts from sheep seminal vesicles and human seminal fluid, prostaglandins have been found to be produced by most mammalian tissues.

Activities of different prostaglandins include stimulation or relaxation of smooth muscle, dilation of small arteries, bronchial dilation, lowering of blood pressure, inhibition of gastric secretion, lipolysis and platelet aggregation, and induction of labor, abortion and menstruation.

It is becoming increasingly evident that PGs reduce intraocular pressure by increasing uveoscleral outflow. This is true for both the F type and A type of PGs and, hence presumably also for the E and B type PGs. See also, U.S. Patent US-B-4,599,353, issued July 8, 1986 and EP-A-242.580.

Contraction of the ciliary muscle as induced by pilocarpine, for example, reduces or blocks uveoscleral outflow while uveoscleral outflow may be assumed to be increased by relaxation of the ciliary muscle. This is because the pathway of uveoscleral outflow is through the muscle part of the ciliary body, i.e., relaxation of the muscle increases the extracellular space between muscle fibers in this tissue, whereas contraction of the muscle decreases this space, thus decreasing or eliminating this flow pathway.

PGs could relax the ciliary muscle by one of two mechanisms: either by causing the release of catecholamines from adrenergic nerve endings contained within the ciliary muscle; or by acting on the ciliary muscle directly, causing relaxation by interaction with PG receptors of the muscle fiber surface. In the former case, adrenergic blocking agents, particularly beta blockers, would block the PG-induced increase in uveoscleral outflow. In the latter case, beta blockers would not block the beneficial intraocular pressure reducing effects of PGs.

Therefore, if topically applied, PGs would reduce intraocular pressure by relaxing the ciliary muscle through the release of catecholamines; combined therapy of an adrenergic blocking agent and PGs would be counterproductive since the adrenergic blocking agent would block the ocular hypotensive effect of the PG. However, if PGs acted directly on the ciliary muscle without the mediation of catecholamines, than adrenergic blocking agents would not interfere with the ocular hypotensive effect of PGs.

The most effective way of reducing intraocular pressure is by affecting both sides of the pressure equation: $P=F\times R$ ; where R is the resistance to the outflow of aqueous humor, F is flow (which, in turn, equals the rate of secretion of aqueous humor) and P equals the effective pressure gradient across the site of resistance (namely, intraocular pressure - episcleral venous pressure). According to currently accepted concepts, there are two sites of aqueous humor outflow from the eye; the conventional outflow through the trabecular meshwork and the above-mentioned uveoscleral flow through the ciliary muscle. Pilocarpine decreases the resistance in the conventional flow channels through the trabecular meshwork; therefore, it works effectively in combination with an adrenergic blocking agent. However, pilocarpine at the same time decreases uveoscleral outflow by contracting the ciliary muscle. In some cases of glaucoma, when flow through the trabecular meshwork is reduced to the point when it cannot be effectively increased by pilocarpine, pilocarpine can have only a very small additional beneficial effect or may have an adverse rather than a beneficial effect by reducing the remaining outflow channels through the ciliary muscle. Therefore, a combination drug, or drug regimen, that acts by decreasing aqueous humor secretion, such as an adrenergic blocking agent and a PG would be ideal, provided that the adrenergic blocking agent can be shown not to block the ocular hypotensive effects of PGs.

In the medical treatment of glaucoma, combination therapy, therefore, is commonly required since in many cases effective intraocular pressure control cannot be maintained with a single drug. The experiments set forth herein establish that the use of a combination of an adrenergic blocking agent and a PG have a great advantage from a physiological point of view, since they show that adrenergic blocking agents do not block the ocular hypotensive effect of a PG and since these blocking agents act by reducing the secretion of aqueous humor whereas PGs, as stated above, act by increasing uveoscleral outflow. In addition, using a combination of an adrenergic blocking agent and a prostaglandin, each or them in a concentration lower than would be required if used separately in the treatment or ocular hypertension and glaucoma, would yield a significant reduction in the occurrence of such side effects as ocular discomfort, irritative responses, conjunctival hyperemia, and cardiovascular response.

Summary Of The Invention

This invention provides a composition for the topical treatment of ocular hypertension or glaucoma which comprises an effective intraocular pressure reducing amount of a mixture of a beta-adrenergic blocking agent and a prostaglandin of type A, E or F in an ophthalmically compatible carrier.

Brief Description Of The Figures

Figure 1 This figure shows the effect of a 2% timolol solution and a combination of a 2% timolol solution and 0.5 µg $PGF_{2\alpha}$-1-isopropyl ester on the intraocular pressure in cat eyes. Application of 0.5 µg $PGF_{2\alpha}$-1-isopropyl ester to eyes already treated with a 2% timolol solution caused a significant further drop in intraocular pressure within one hour.

Figure 2 This figure shows the ocular hypotensive effect of a solution containing both timolol (at a concentration of 2.0%) and $PGF_{2\alpha}$-1-isolpropyl ester (at a final concentration of 0.002%) on cat eyes as compared to the ocular hypotensive effect of timolol applied by itself.

Figure 3 This figure snows the ocular hypotensive effect of $PGF_{2\alpha}$-1-isopropyl ester (at a final concentration of 0,002%) on cat eyes pretreated 2 hrs earlier with an 0.5% solution of betaxolol.

Figure 4 This figure shows the ocular hypotensive effect of $PGF_{2\alpha}$-1-isopropyl ester on cat eyes pretreated 2 hrs earlier with an 0.5% solution of levobunolol.

Detailed Description Of The Invention

This invention provides a composition comprising an effective intraocular pressure reducing amount of a mixture of a beta-adrenergic blocking agent and a prostaglandin of type A, E or F in an ophthalmically compatible carrier, so as to reduce the intraocular pressure of the eye and maintain such reduced intraocular pressure.

As the adrenergic blocking agent there is employed in the practice of this invention a beta blocker. The presently preferred beta blockers or beta-adrenergic blocking agent, respectively are timolol maleate, betaxolol hydrochloride, and levobunolol hydrochloride. Of the prostaglandins of the A, E or F type employed in the practice of this invention, the presently preferred ones are $PGF_{2\alpha}$ or a $PGF_{2\alpha}$ derivative, specifically $PGF_{2\alpha}$-1-isopropyl ester.

Additionally, a range of concentrations of beta-adrenergic blocking agents may be employed in the practice of the invention. However, the preferred amount present in the mixture is from 0.01 µg to 1,000 µg, specifically from about 5 µg to 500 µg.

The presently preferred effective amount of prostaglandin present in the mixture is from 0.01 µg to 1,000 µg, specifically from 0.1 µg to 50 µg.

The ophthalmically compatible carrier may be any well known carrier. Presently preferred for use in the practice of this invention are aqueous solutions, such as a saline solution containing an ophthalmically compatible preservative a surfactant, and an agent, such as a soluble polymer, to increase the viscosity of the solution. In a preferred embodiment, the mixture is dissolved in the ophthalmically compatible carrier.

Various regimens may be employed applying the present composition for treating ocular hypertension or glaucoma in the subject's eye. In the preferred embodiment, the composition is contacted with the surface of the eye, i.e., the cornea, periodically, preferably at least daily, to reduce intraocular pressure and maintain such reduced intraocular pressure.

This invention particularly provides a composition for the topical treatment of ocular hypertension or glaucoma comprising an effective intraocular pressure reducing amount of a mixture of levobunolol hydrochloride and $PGF_{2\alpha}$-1-isopropyl ester dissolved in an ophthalmically compatible carrier.

EXPERIMENTAL RESULTS

Materials

The following materials used in the practice of this invention may be obtained from commercial sources: timolol maleate, (as Timoptic™ from Merck Sharp & Dohme Division of Merck & Co. Inc., West Point, PA), betaxolol hydrochloride (as Betoptic from Alcon Laboratories, Fort Worth, Texas), and levobunolol HCl (as Betagan™ from Allergan America, Hormigueros, Puerto Rico).

Method

Trained, unanesthetized cats (1.5 to 3.0 kg) which showed on biomicroscopical examination of evidence of ocular inflammation such as anterior chamber flare or cellular invasion were used in all experiments. Eight cats were used in each set of experiments.

Intraocular pressures (IOP) were measured using an Alcon floating tip Applanation Pheumatonograph. The horizontal width of the pupil was measured using a millimeter pupil gauge. IOP and pupil diameters were measured before and several times after drug applications. Biomicroscopical examination of the anterior chamber was performed before and at 3, 6 and 24 hours after PG application.

$PGF_{2\alpha}$-1-isopropyl ester ($PGF_{2\alpha}$-IE) was supplied by Pharmacia AB (Uppsala, Sweden) already dissolved in 0.5% polysorbate 80 in normal saline containing 0.01% benzalkonium chloride; all dilutions were made up in this vehicle solution.

Timolol maleate powder was obtained from Merck, Sharp and Dohme (West Point, PA) and was dissolved in 0.5% polysorbate 80 in normal saline with 0.01% benzalkonium chloride (40 mg/ml, and 20 mg/ml, respectively). Betaxolol hydrochloride (Betoptic; Alcon, Fort Worth, TX) at a concentration of 0.5% (5 mg/ml) and levobunalol hydrochloride (Betagan; Allergan, Irvine, CA) at a concentration of 0.5% (5 mg/ml), were obtained from the pharmacy.

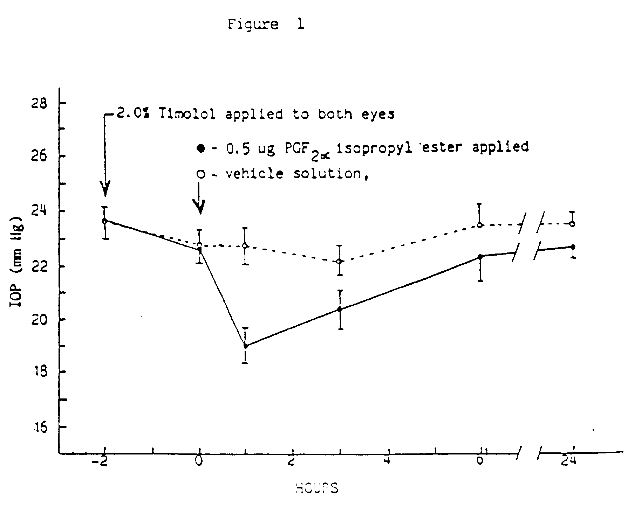

In most experiments (those shown in Figures 1, 3 and 4), one beta-adrenergic blocking agent (500 µg Betoptic, or 125 µg Betagen) in a volume of 25 µl was applied first to both eyes of each cat immediately after baseline IOP and pupil diameter measurements. After 2 hours, immediately following another IOP and pupil diameter measurement, 0.5 µg (0,002%) of $PGF_{2\alpha}$-IE in a volume of 25 µl was applied to one eye of each cat while the contralateral eye received 25 µl of the vehicle solution. IOP and pupil diameter measurements along with biomicroscopic examination of the anterior chamber were performed as described above.

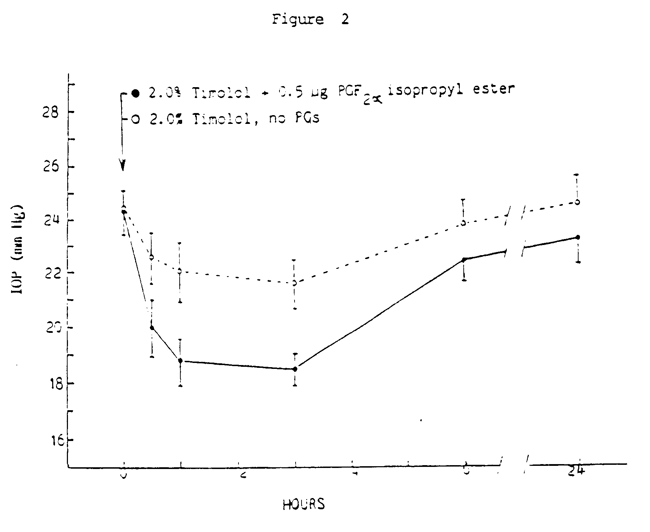

In one group of 8 cats (results shown in Figure 2), a 25 µl-aliquot of a mixture of equal volumes of 4% timolol and 0,004% $PGF_{2\alpha}$-IE yielding a dose of 500 µg of timolol and 0.5 µg of $PGF_{2\alpha}$-IE in each 25 µl of 2% timolol (500 µg) . IOP and pupil diameter measurements were taken as above.

Discussion

The beta blocker, timolol maleate, did not block the ocular hypotensive effect of $PGF_{2\alpha}$-1-isopropyl ester. As shown in Figure 1, when a 2% timolol solution made up in an appropriate aqueous vehicle, was applied to both eyes of cats, there was some intraocular pressure reduction in both eyes at 2 hours. However, in the eyes which were then treated with 0.5 µg of $PGF_{2\alpha}$-1-isopropyl ester (which is a threshold ocular hypotensive dose of $PGF_{2\alpha}$ in this species), a highly significant further drop in intraocular pressure occured within one hour. In the contralateral eyes that received only timolol 2 hours earlier, the intraocular pressure only showed a very small, further decrease as compared to the pressure decrease observed during the first 2 hours.

Furthermore, as shown in Figure 2, there was a significant pressure reduction in cat eyes that were treated with 25 µl of the vehicle solution containing both timolol maleate (at a final concentration of 2.0%) and $PGF_{2\alpha}$-1-isopropyl ester (at a final concentration of 0.002%) as compared to eyes that were treated with an identical volume of solution containing only timolol maleate. It should be noted that adrenergic receptors in cats have the same characteristics as in other species, and , therefore, timolol maleate must be regarded as an adrenergic blocking agent in that species. Timolol maleate, however, is well-known to be a less effective pressure-reducing agent in cats than in humans. Therefore, in the human, the combination of timolol and a PG must have a greater effect than in cats. Since timolol maleate in the human, as in cats, acts by reducing the rate of aqueous humor production, while PG acts by increasing uveoscleral outflow, as long as timolol does not block the relaxing effect of PGs on the ciliary muscle, the effects of these two drugs must be at least additive.

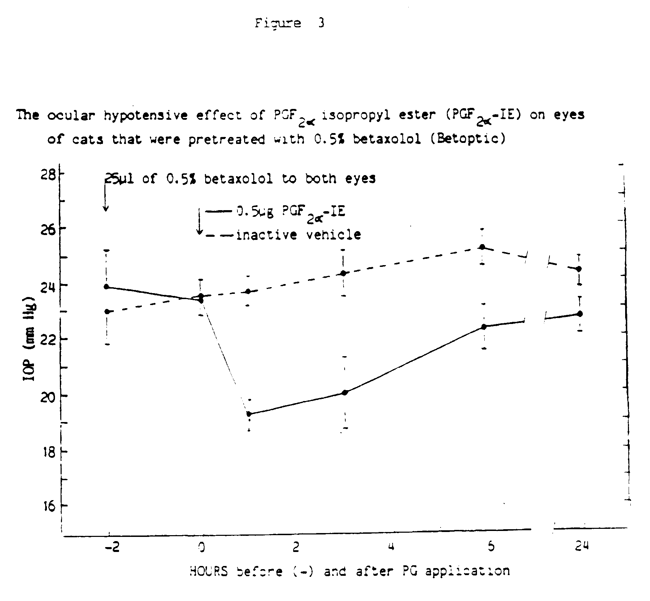

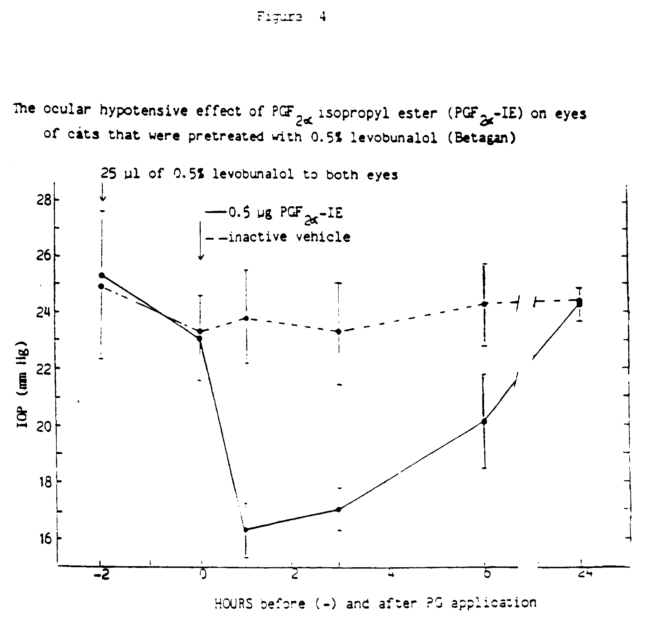

Betaxolol hydrochloride and levobunolol hydrochloride also were employed in the practice of this invention, using 25 µl of the 0.5% clinically used solutions of Betoptic[®] and Betagen[®], respectively. As is shown in Figure 3 and Figure 4, neither betaxolol hydrochloride nor levobunolol hydrochloride blocked the ocular hypotensive effect of $PGF_{2\alpha}$-1-isopropyl ester. Thus levobunolol hydrochloride may be a very good candidate for combined therapy, since there was a more pronounced reduction of intraocular pressure within one hour after the topical application of 0.5 µg of $PGF_{2\alpha}$-1-isopropyl ester in eyes that were pretreated with this beta blocker than typically occurs with this dose of $PGF_{2\alpha}$-1-isopropyl ester. See, for example, the much smaller ocular hypotensive response to this dose of $PGF_{2\alpha}$-1-isopropyl ester in betaxolol hydrochloride pretreated eyes (Figure 3) as compared to levobunolol hydrochloride pretreated eyes (Figure 4).

The positive interaction between levobunolol hydrochloride and $PGF_{2\alpha}$-1-isopropyl ester suggests that $PGF_{2\alpha}$-1-isopropyl ester may yield an effective intraocular pressure reduction at $PGF_{2\alpha}$-1-isopropyl ester doses even less than 0.5 µg. While 0.5 µg is already a very small dose of $PGF_{2\alpha}$-1-isopropyl ester, a further reduction in this dose may have important clinical significance, since studies of Alm and Villumsen (Proceeding of the International Society for Eye Research, Vol. IV, #18:15, Seventh International Congress of Eye Research, Nagoya, Japan 1986) on human eyes show that reduction of the $PGF_{2\alpha}$-1-isopropyl ester dose from 2.5 µg to 0.5 µg is sufficient to cause a considerable reduction in side effects, primarily conjunctival hyperemia. Therefore, the reduction of the $PGF_{2\alpha}$-1-isopropyl ester dose in a combined therapy with levobunolol, or some other beta blocker will yield a significant reduction of the undesirable side effects of $PGF_{2\alpha}$-1-isopropyl ester.

**Claims**

1. A composition for the topical treatment of ocular hypertension or glaucoma comprising an effective intraocular pressure reducing amount of a mixture of a beta-adrenergic blocking agent and a prostaglandin of type A, E, or F, in an ophthalmically compatible carrier.

2. A composition of claim 1, wherein the beta-adrenergic blocking agent is timolol maleate.

3. A composition of claim 1, wherein the beta-adrenergic blocking agent is betaxolol hydrochloride.

4. A composition of claim 1, wherein the beta-adrenergic blocking agent is levobunolol hydrochloride.

5. A composition of claim 1, wherein the prostaglandin of type F is prostaglandin $F_{2\alpha}$ or an ester of prostaglandin $F_{2\alpha}$.

6. A composition of claim 5, wherein the ester of prostaglandin $F_{2\alpha}$ is $PGF_{2\alpha}$-1-isopropyl ester.

7. A composition of claim 1, wherein the beta-adrenergic blocking agent is present in the mixture in an amount from between 0.01 µg and 1,000 µg.

8. A composition of claim 7, wherein the beta-adrenergic blocking agent is present in the mixture in an amount from between 0.01 µg and 500 µg.

9. A composition of claim 1, wherein the prostaglandin of type A, E, or F is prosent in the mixture in an amount between about 0.01 µg and 1,000 µg.

10. A composition of claim 9, wherein the prostaglandin of type A, E, or F is present in the mixture in an amount between about 0.1 µg and 50 µg.

11. A composition of claim 1, wherein the ophthalmically compatible carrier comprises an aqueous solution.

12. A composition of claim 11, wherein the aqueous solution is a saline solution containing an ophthalmically compatible preservative, a surfactant, and an agent, such as a soluble polymer, to increase the viscosity of the solution.

13. A composition of claim 1, wherein the mixture is dissolved in the ophthalmically compatible carrier.

14. A composition for the topical treatment of ocular hypertension or glaucoma comprising an effective intraocular pressure reducing amount of a mixture of levobunolol hydrochloride and $PGF_{2\alpha}$-1-isopropyl ester dissolved in an ophthalmically compatible carrier.

15. The use of a beta-adrenergic blocking agent and a prostaglandin of type A, E, or F for the manufacture of a composition for the topical treatment of ocular hypertension or glaucoma.

16. The use of claim 15, wherein the beta-adrenergic blocking agent is timolol maleate.

17. The use of claim 15, wherein the beta-adrenergic blocking agent is betaxolol hydrochloride.

18. The use of claim 15, wherein the beta-adrenergic blocking agent is levobunolol hydrochloride.

19. The use of claim 15, wherein the prostaglandin of type F is prostaglandin $F_{2\alpha}$ or an ester of prostaglandin $F_{2\alpha}$.

20. The use of claim 19, wherein the ester of prostaglandin $F_{2\alpha}$ is $PGF_{2\alpha}$-1-isopropyl ester.

21. The use claim 16, wherein the beta-adrenergic blocking agent is admixed in an amount from between 0.01 µg and 1,000 µg.

22. The use of claim 21, wherein the beta-adrenergic blocking agent is admixed in an amount from between 0.01 µg and 500 µg.

23. The use of claim 15, wherein the prostaglandin of type A, E, or F is admixed in an amount between about 0.01 µg and 1,000 µg.

24. The use of claim 23, wherein the prostaglandin of type A, E, or F is admixed in an amount between about 0.1 µg and 50 µg.

25. The use of levobunolol hydrochloride and $PGF_{2\alpha}$-1-isopropyl ester for the manufacture of a composition for the topical treatment of ocular hypertension or glaucoma.

**Patentansprüche**

1. Mittel zur topischen Behandlung von Augenüberdruck oder Glaukom, enthaltend eine zur Verminderung des Augeninnendrucks wirksame Menge eines Gemischs aus einem adrenergischen β-Blocker und einem Prostaglandin vom A-, E- oder F-Typ in einem augenverträglichen Träger.

2. Mittel nach Anspruch 1, dadurch gekennzeichnet, daß es sich bei dem adrenergischen β-Blocker um Timololmaleat handelt.

**3.** Mittel nach Anspruch 1, dadurch gekennzeichnet, daß es sich bei dem adrenergischen β-Blocker um Betaxololhydrochlorid handelt.

**4.** Mittel nach Anspruch 1, dadurch gekennzeichnet, daß es sich bei dem adrenergischen β-Blocker um Levobunololhydrochlorid handelt.

**5.** Mittel nach Anspruch 1, dadurch gekennzeichnet, daß es sich bei dem Prostaglandin des F-Typs um Prostaglandin $F_{2\alpha}$ oder einen Ester von Prostaglandin $F_{2\alpha}$ handelt.

**6.** Mittel nach Anspruch 5, dadurch gekennzeichnet, daß es sich bei dem Ester des Prostaglandins $F_{2\alpha}$ um $PGF_{2\alpha}$-1-Isopropylester handelt.

**7.** Mittel nach Anspruch 1, dadurch gekennzeichnet, daß der adrenergische β-Blocker in dem Gemisch in einer Menge zwischen 0,01 µg und 1000 µg enthalten ist.

**8.** Mittel nach Anspruch 7, dadurch gekennzeichnet, daß der adrenergische β-Blocker in dem Gemisch in einer Menge zwischen 0,01 µg und 500 µg enthalten ist.

**9.** Mittel nach Anspruch 1, dadurch gekennzeichnet, daß das Prostaglandin des A-, E- oder F-Typs in dem Gemisch in einer Menge zwischen etwa 0,01 µg und 1000 µg enthalten ist.

**10.** Mittel nach Anspruch 9, dadurch gekennzeichnet, daß das Prostaglandin des A-, E- oder F-Typs in dem Gemisch in einer Menge zwischen etwa 0,1 µg und 50 µg enthalten ist.

**11.** Mittel nach Anspruch 1, dadurch gekennzeichnet, daß der augenverträgliche Träger aus einer wäßrigen Lösung besteht.

**12.** Mittel nach Anspruch 11, dadurch gekennzeichnet, daß es sich bei der wäßrigen Lösung um eine physiologische Kochsalzlösung mit einem augenverträglichen Konservierungsmittel, einem Netzmittel und einem Mittel, z.B. einem löslichen Polymerisat zur Erhöhung der Viskosität der Lösung handelt.

**13.** Mittel nach Anspruch 1, dadurch gekennzeichnet, daß das Gemisch in dem augenverträglichen Träger gelöst ist.

**14.** Mittel zur topischen Behandlung von Augenüberdruck oder Glaukom, enthaltend eine zur Verminderung des Augeninnendrucks wirksame Menge eines Gemischs aus Levobunololhydrochlorid und $PGF_{2\alpha}$-1-Isopropylester in Form einer Lösung in einem augenverträglichen Träger.

**15.** Verwendung eines adrenergischen β-Blockers und eines Prostaglandins vom A-, E- oder F-Typ zur Herstellung eines Mittels zur topischen Behandlung von Augenüberdruck oder Glaukom.

**16.** Verwendung nach Anspruch 15, wobei der adrenergische β-Blocker aus Timololmaleat besteht.

**17.** Verwendung nach Anspruch 15, wobei der adrenergische β-Blocker aus Betaxololhydrochlorid besteht.

**18.** Verwendung nach Anspruch 15, wobei der adrenergische β-Blocker aus Levobunololhydrochlorid besteht.

**19.** Verwendung nach Anspruch 15, wobei das Prostaglandin vom F-Typ aus Prostaglandin $F_{2\alpha}$ oder einem Ester von Prostaglandin $F_{2\alpha}$ besteht.

**20.** Verwendung nach Anspruch 19, wobei es sich bei dem Ester von Prostaglandin $F_{2\alpha}$ um $PGF_{2\alpha}$-1-Isopropylester handelt.

**21.** Verwendung nach Anspruch 16, wobei der adrenergische β-Blocker in einer Menge zwischen 0,01 µg und 1000 µg zugemischt ist.

**22.** Verwendung nach Anspruch 21, wobei der adrenergische β-Blocker in einer Menge zwischen 0,01 µg und 500 µg zugemischt ist.

**23.** Verwendung nach Anspruch 15, wobei das Prostaglandin vom A-, E- oder F-Typ in einer Menge zwischen etwa 0,01 µg und 1000 µg zugemischt ist.

**24.** Verwendung nach Anspruch 23, wobei das Prostaglandin vom A-, E- oder F-Typ in einer Menge zwischen etwa 0,1 µg und 50 µg zugemischt ist.

**25.** Verwendung von Levobunololhydrochlorid und $PGF_{2\alpha}$-1-Isopropylester zur Herstellung eines Mittels zur topischen Behandlung von Augenüberdruck oder Glaukom.

**Revendications**

**1.** Composition pour le traitement topique de l'hypertension oculaire ou du glaucome, comprenant une quantité efficace pour réduire la pression intraoculaire d'un mélange d'un β-adrénolytique et d'une prostaglandine de type A, E ou F, dans un véhicule acceptable du point de vue ophtalmique.

**2.** Composition selon la revendication 1, dans laquelle le β-adrénolytiqueest le maléate de timolol.

**3.** Composition selon la revendication 1, dans laquelle le β-adrénolytiqueest le chlorhydrate de betaxolol.

4. Composition selon la revendication 1, dans laquelle le β-adrénolytiqueest le chlorhydrate de levobunolol.

5. Composition selon la revendication 1, dans laquelle la prostaglandine de type F est la prostaglandine $F_{2\alpha}$ ou un ester de prostaglandine $F_{2\alpha}$.

6. Composition selon la revendication 5, dans laquelle l'ester de prostaglandine $F_{2\alpha}$ est le 1-isopropylester de $PGF_{2\alpha}$.

7. Composition selon la revendication 1, dans laquelle le β-adrénolytiqueest présent dans le mélange en une quantité comprise entre 0,01 µg et 1 000 µg.

8. Composition selon la revendication 7, dans laquelle le β-adrénolytiqueest présent dans le mélange en une quantité comprise entre 0,01 µg et 500 µg.

9. Composition selon la revendication 1, dans laquelle la prostaglandine de type A, E ou F est présente dans le mélange en une quantité comprise entre environ 0,01 µg et 1 000 µg.

10. Composition selon la revendication 9, dans laquelle la prostaglandine de type A, E ou F est présente dans le mélange en une quantité comprise entre environ 0,1 µg et 50 µg.

11. Composition selon la revendication 1, dans laquelle le véhicule acceptable du point de vue ophtalmique comprend une solution aqueuse.

12. Composition selon la revendication 11, dans laquelle la solution aqueuse est une solution salée contenant un conservateur acceptable du point de vue ophtalmique, un tensioactif et un agent, tel qu'un polymère soluble, pour augmenter la viscosité de la solution.

13. Composition selon la revendication 1, dans laquelle le mélange est dissous dans le véhicule acceptable du point de vue ophtalmique.

14. Composition pour la traitement topique de l'hypertension oculaire ou ou glaucome, comprenant une quantité efficace pour réduire la pression intra-oculaire d'un mélange de chlorhydrate de levobunolol et de 1-isopropylester de $PGF_{2\alpha}$ dissous dans un véhicule acceptable du point de vue ophtalmique.

15. Utilisation d'un β-adrénolytique et d'une prostaglandine de type A, E ou F pour la préparation d'une composition pour le traitement topique de l'hypertension oculaire ou du glaucome.

16. Utilisation selon la revendication 15, dans laquelle le β-adrénolytique est le maléate de timolol.

17. Utilisation selon la revendication 15, dans laquelle le β-adrénolytique est le chlorhydrate de betaxolol.

18. Utilisation selon la revendication 15, dans laquelle le β-adrénolytique est le chlorhydrate de levobunolol.

19. Utilisation selon la revendication 15, dans laquelle la prostaglandine de type F est la prostaglandine $F_{2\alpha}$ ou un ester de prostaglandine $F_{2\alpha}$.

20. Utilisation selon la revendication 19, dans laquelle l'ester de prostaglandine $F_{2\alpha}$ est le 1-isopropylester de $PGF_{2\alpha}$.

21. Utilisation selon la revendication 16, dans laquelle le β-adrénolytique est mélangé en une quantité comprise entre 0,01 µg et 1 000 µg.

22. Utilisation selon la revendication 21, dans laquelle le β-adrénolytique est mélangé en une quantité comprise entre 0,01 µg et 500 µg.

23. Utilisation selon la revendication 15, dans laquelle la prostaglandine de type A, E ou F est mélangée en une quantité comprise entre environ 0,01 µg et 1 000 µg.

24. Utilisation selon la revendication 23, dans laquelle la prostaglandine de type A, E ou F est mélangée en une quantité comprise entre environ 0,1 µg et 50 µg.

25. Utilisation du chlorhydrate de levobunolol et du 1-isopropylester de $PGF_{2\alpha}$ pour la préparation d'une composition pour le traitement topique de l'hypertension oculaire ou du glaucome.

Figure 1

Figure 2

Figure 3

The ocular hypotensive effect of PGF$_{2\alpha}$ isopropyl ester (PGF$_{2\alpha}$-IE) on eyes
of cats that were pretreated with 0.5% betaxolol (Betoptic)

28  25μl of 0.5% betaxolol to both eyes

— 0.5μg PGF$_{2\alpha}$-IE
— — inactive vehicle

IOP (mm Hg)

HOURS before (-) and after PG application

10

Figure 4

The ocular hypotensive effect of $PGF_{2\alpha}$ isopropyl ester ($PGF_{2\alpha}$-IE) on eyes of cats that were pretreated with 0.5% levobunalol (Betagan)

25 µl of 0.5% levobunalol to both eyes

——0.5 µg $PGF_{2\alpha}$-IE

– –inactive vehicle

IOP (mm Hg)

HOURS before (-) and after PG application